# EUROPEAN PATENT APPLICATION

(11) **EP 4 681 763 A1**
(43) Date of publication of application: **21.01.2026**
(21) Application number: 25181623.7
(22) Date of filing: 10.06.2025
(51) Int. Cl.: A61M 21/00

(54) **A DEVICE OF DEVELOPMENT OF CONCENTRATION COMPRISING THREE MODES AND A COMPUTER-IMPLEMENTED METHOD USED IN THE SAME**

(30) Priority: 10.06.2024 GR 20240100429
(71) Applicant: Grabovoi, Grigorii Petrovich, Belgrad, 11102 (RS)
(72) Inventor: Grabovoi, Grigorii Petrovich, Belgrad, 11102 (RS)
(74) Representative: Fenix Legal KB

(57) **Abstract**

A device 100 for development of concentration, the device 100 comprising:
an optical sensing unit, the optical sensing unit comprising a plurality of one or more lenses 201, 202 capable of holding sensitive elements, wherein the plurality of sensitive elements are configured to sense a biological signal provided by a user in at least three operational modes, three switches for switching between the plurality of operation modes; a plurality of lightning units configured to indicate each of the plurality of operation modes by emitting a predetermined light signal; a processing unit for processing information using artificial intelligence.

## Description

### TECHNICAL FIELD

The present invention relates generally to optical devices and computer-implemented methods in a device for developing concentration and can be used in various applications including education, training and therapeutic settings.

### BACKGROUND

There is a variety of devices that for instance use laser beams as communication channels between the transmitter and a receiver of signals. Each transmitting signal is then generated by a laser generator with a device for modulating the laser beam with a data signal connected to a source of data signals. Each receiving signal is received by a photo detector and a device for converting the perceived laser modulated radiation into electrical data signals.

A disadvantage of this kind of known data transmission system is its low operational reliability, due to the complexity of the system design including a large number of complex signal transmitters and receivers with multifunctional connection and complex precision guidance system with moving elements. In a known system, when transmitting information between a transmitter and a signal receiver located at considerable distances from each other, for example, when transmitting information over hundreds or thousands kilometres using several repeaters, the delay in transmitting information can be tenths of a second. Such a known system has insufficiently high noise immunity, since when any obstacle appears on the laser communication line, interference occurs in the operation of the system or disruption of transmitted signals.

It is therefore important to focus on the principle of similarity. The principle of similarity is based on the theory of wave synthesis in combination with the unified reality theory (see Ph.D. Thesis in Physical and Mathematical Sciences, G.P. Grabovoi, "Research and Analysis of Fundamental Definitions of Optical Systems for Prediction of Industrial Nature Earthquakes and Disasters", Moscow, RAEN publishing House, 1999, pp.9-19. The below mentioned methods are further based on physical and mathematical theories, experimental results, physical and mathematical calculations, and the results of these calculations set forth in the publication titled "Research and Analysis of the Fundamental Definitions of Optical Systems in Disaster Prevention and Predictive Microprocessor Control", "Electronic Equipment, Series 3, Microelectronics", 1999, edition 1 (153), and other scientific materials. There are as well two other patent documents forming the ground to the claimed device and claimed method: RU2148845C1 titled "Method of Prevention of Catastrophes and Equipment for its realization", published on 10 May, 2000 relating to an optical system incorporating components produced from crystals distributed along direction of propagation of emission and positioned in glass sphere for anticipating catastrophe in a zone; and RU2163419C1 titled "Data transmission System", published on 20 February, 2001 based on the principle of similarity and relating to a data transmission system having a signal transmitter including spherical glass sensing elements and a signal receiver mounted at certain distance from transmitter having a spherical module spaced apart from the latter for improving operating reliability and noise immunity of the system.

US 12,144,599 B2 'DEVICE OF DEVELOPMENT OF CONCENTRATIONS OF ETERNAL LIFE PRK-1U IS OF THREE-MODES', published on November 19, 2024, related to a device for the development of concentration comprising an optical sensor unit with multiple sensitive elements configured to receive a signal submitted by the user;

This invention application is essentially identical to the invention application filed by the same applicant Grigorii Petrovich Grabovoi in the Greek Organization for Industrial Property (OBI), which received a decision on the issuance of the patent application 20240100429 on the basis of the final report of the examination of the invention with the end date of May 5, 2025.

This invention application is essentially identical to the application filed by the same applicant, Grigorii Petrovich Grabovoi, with the German Patent and Trademark Office (DPMA) as a utility model, registered on December 13, 2024, under utility model number DE 20 2024 103 073 U1 titled 'Ein Gerät zur Entwicklung der Konzentration mit drei Mode'; The PRK-1UM device has been registered as an industrial design in the following countries:
90582-01 (Benelux: Belgium, Netherlands, Luxembourg); 6406099 (United Kingdom); 148367 (Switzerland); 1790930 (Japan); 202418610 (Australia).

### SUMMARY OF THE INVENTION

By deviating from the above-mentioned logical and widely accessible way of solving the problem, makes the constitution of the present disclosure both unique and relevant. The present disclosure meets all the criteria listed above.

The objective of the present invention is to provide a device for development concentration of a user by using an information transmission system which increase the operational reliability and at the same time ensures the transmission of information without delays and immune to noise. Further, the concentration of a user may be defined as a mental strength of a person concentrating on a specific goal, e.g. on an eternal life.

The present disclosure overcomes all the above-mentioned problems by providing a device, method and computer-implemented method for development of concentration of a user. The device is a three-mode device, may also be referred as device of concentration on eternal life PRK-1UM three-mode device.

There is provided a device for development of concentration, the device comprising: an optical sensing unit, the optical sensing unit comprising a plurality of one or more lenses capable of holding sensitive elements, wherein the plurality of sensitive elements are configured to sense a biological signal provided by a user in at least three operational modes, the signal being associated with a plurality of electromagnetic fields; and an outgoing signal obtained based on the biological signal and the plurality of electromagnetic fields; an optical emitting unit configured to emit the outgoing signal; wherein the optical emitting unit emits the outgoing signal in a form of at least an optical signal; three switches for switching between the plurality of operation modes; a plurality of lightning units configured to indicate each of the plurality of operation modes by emitting a predetermined light signal; and further comprising: at least two lasers placed inside the device, wherein a first laser is constantly turned on during a second operation mode representing the emittance of a static light signal by one of the plurality of the lightning units; the second laser being connected to a motion sensor capable of turning on and off when a user is in a range proximity and representing the emittance of a repetitively-pulsed light signal by another lightning unit; and a processing unit for processing information from at least one of a motion sensor, a SD-card, a laser, a DC/DC converter, a switch selector, and a USB-adapter by using artificial intelligence.
Preferably, the device may comprise a power source in communication with the optical sensing unit and the optical emitting unit. The plurality of sensitive elements may be spherical. The device may comprise a housing and a lid and there may be a plurality of numbers or letters placed on one of the housing and the lid, wherein the numbers or letters are symbols for focusing concentration of the user. The first set numbers may include digits 1, 4 and 5 and a second set of numbers includes digits 2, 7, 8 and 9, 0, 6, 3.

Preferably, the one or more lenses may be disposed on the lid.

Further, the device may comprise a converting unit configured to convert the outgoing signal into an electrical signal.

Further, the device may comprise a SD-card adapter. In accordance with the wave synthesis process, an SD-card adapter may be installed for realizing the transition of an electron to an infinite medium through a number on the display. The third operation mode, due to the operation of the artificial intelligence may therefore require the use of an SD-card. The concentration on the numbers read from the SD-card when monitored on a display will allow a user to simulate the operation of the third operation mode. Thereby, by comparing the operation of the third operation mode and the simulated operation of the third operation mode, the user will be able to accelerate the development of concentration and strengthening the concentration of mental models of events.

Further, an OLED display may monitor the number series read from a SD-card inserted in the SD-card adapter.

Further, there may be provided a compass installed on the lid for directing the laser beams in a specific direction.

Further, there may be provided a USB connector installed on the rear of the device for connecting for example external power to the device.

Further, there may be provided LED-lights for displaying number series from a SD-card in the form of light pulses.

Another object of the present invention is providing a method for development of concentration, the method comprising the steps of: providing one or more lenses for focusing concentration of a user, the one or more lenses being part of an optical sensing unit, the optical sensing unit comprised in the device; switching between a plurality of operation modes using three switches; indicating, by a lightning unit, each of the plurality operation modes by emitting a predetermined light signal; sensing, by a plurality of sensitive elements of the optical sensing unit, a biological signal provided by a user in at least three operational modes, the signal being associated with a plurality of electromagnetic fields; and obtaining an outgoing signal based on the biological signal and the plurality of electromagnetic fields; converting the biological signal by a converting unit to obtain an outgoing signal; processing the outgoing signal by a processing unit to obtain an optical signal generated by an optical emitting unit.

Preferably, the method may comprise the step of providing a power source, wherein the power source is in communication with the optical sensing unit and the optical emitting unit.

Preferably, the optical emitting unit may include an optical lens.
Another third object of the present invention is to provide a computer-implemented method of developing concentration loadable into the claimed device, comprising the steps of: receiving an electrical signal converted by a converting unit from at least an outgoing signal; the outgoing signal is obtained from the optical sensing unit upon a biological signal associated with a plurality of electromagnetic fields provided by a user; processing the electrical signal by a processing unit using artificial intelligence for outputting an optical signal generated by the optical emitting unit associated with the concentration of a user.

### DESRICPTION OF FIGURES

In the following, the disclosure will be described in further detail with references to the exemplary device, methods and systems in the drawings, in which:
FIG 1. shows a top left front side view of a device according to a first object of the present disclosure.
FIG 2. shows a top view of the device according to the first object of the present disclosure.
FIG 3. shows an internal view of the first object of the present disclosure.
FIG 4. shows an overview of the components of the first object of the present disclosure.
FIG 5. shows a block scheme of the first object of the present disclosure.
FIG 6. shows a computing system adapted to a computer-implemented method according to a third object of the present disclosure.
FIG 7. shows an electrical scheme of the first object of the present disclosure.
FIG 8. shows a flow chart illustrating a method of the second object of the present disclosure.

### DESCRIPTION OF EMBODIMENT

When concentrating, the user may imagine user consciousness as a sphere of sensitive elements around the user's body supported by the user's body itself. In a next step, the user may imagine that the sphere transforms into a shape similar to the shape of the user's body and then this shape is absorbed into the surface of each sensitive element by the reflection of the light that radiates the body. The user may imagine that the radiation of the body-like shape comes to contact with the surface of this body-like shape spreads to all external infinite space relative to the user's body. The infinite space is considered to be the eternal reality connected with the organism of the user, which results in development of concentration on eternal life.

In accordance with the wave synthesis theory, reality can be considered as a periodic intersection of stationary regions with dynamic regions, while in the intersection zones a synthesis of a dynamic wave and a stationary wave occurs. Any reality phenomenon can be defined in a form of optical systems. Human perception is performed using image-bearing elements of light containing information. In case of transmitting information from a person generating information to be transmitted to an optical sensing element, the person may be considered to be a transmitting optical system. The transmitted information generated by thoughts of the person is received by an optical sensing unit to which the person directs the generated thoughts. As a thought is an electromagnetic wave, it can be transmitted as an element of an optical system. Sensitive elements of the optical sensing unit preferably have the shape of a sphere, as the spherical shape of the sensitive element provides the maximum activation of the sensitive element due to the internal reflection of biological signals. Biological signals or also called biosignals can be generated from electrical, electromagnetic or non-electrical fields and may be brain waves or other type of signals generated by the human body.

The three-mode device for development of concentration performs the detection of the generation of biological signals and electromagnetic fields from electromagnetic waves generated by the user according to the principle of universal connection with control of the purpose of concentration by using artificial intelligence, Al.

In the wave synthesis theory, it is known that a thought generated in a form of radiation simultaneously has two quantum states. The first state is located on one of the sensing elements of a signal transmitter, and the second state is located on a signal receiver. Based on these principles, the device for interacting with thoughts to develop the concentration as described herein was created.

The device works universally to develop the following concentrations to ensure eternal life:
Control 1:
   - Development of concentrations of eternal life for any event.
Control 2:
   - Development of concentrations of eternal life according to controlling clairvoyance.
Control 3:
   - Development of concentrations of eternal life according to control forecasting.
Control 4:
   - Development of eternal life concentrations for rejuvenation. Developing concentrations of eternal life with the help of the present device, in order to master the implemented technologies through spiritual development or controlling clairvoyance.

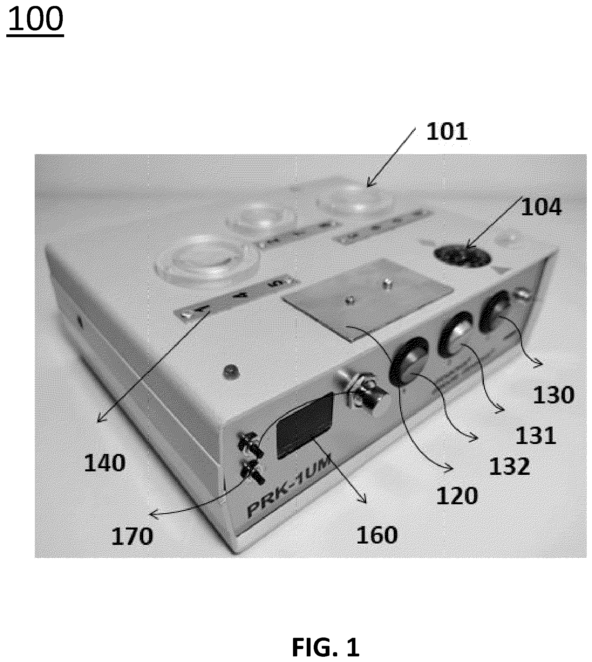

Referring to the figures, FIG. 1 is a general view of a three mode device 100 for development of concentration, hereinafter referred as a device 100. The device may comprise a housing having a substantially rectangular shape. The device shows an exemplary embodiment of the present invention. This embodiment shows three lenses 101 that may be attached to an outer surface of the lid of the device. However, the embodiment requires at least one lens 101 and the other two could therefore be removed. Each of the lenses 101 may be placed in a plate (e.g. metal plate). The diameter of the lenses 101 may be 20mm, 25mm, 60mm, and any other diameter applicable for a particular embodiment of the device 100. The diameter of the plate may be 60mm, 64mm, 70mm, and any other diameter applicable for a particular embodiment of the device 100.

The device may have a further plate 120, 220 for placing stones, such as diamonds, attached to the housing or to the lid of the device.

On the top of the lid of the device there is further at least two lasers, wherein a first laser is constantly turned on when the device is turned on. The second laser is operating in conjunction with a motion sensor for detecting a user less than three meters from the device. However, other types of motion sensors may also be used detecting other ranges.

As seen in Figure 1, symbols as numbers 140 but also as letters may be placed on the cover. For example, the numbers 1, 4, 5 may be placed near a large lens 101 as depicted. The developments of concentration using the presence of figures near the lenses can be made by concentrating on the lenses in a way describe above and adding concentration on the specific symbols.

The device further comprises three switches 132, 131, 130, wherein a first switch 132 is for turning on the device and the operational mode "universal", a light is then turned on. In this embodiment, the switch button for the first operational mode is illuminated red. When the second switch 131 is turned on, the switch button of the second operational mode is illuminated green and the stationary phase of reality is considered to be strengthened. The second mode is manifested by static light emission from a LED-light on the left side of the device.

The last switch button 130 is for turning on the third operational mode which strengthens the dynamic phase of reality (pulse-periodic). However, the first switch mode needs to be switched off in order for the third switch button 130 to be activated. The second switch button 131 starts then flashing and the third switch button 130 is lighted up as blue. The third mode manifests itself by pulsed-periodic light emission from the LED-light on the left side of the device 100. The third switch button 130 enables also additional functions of the device. The third operational mode turns on two lasers, a motion sensor, a SD card adapter or module and an OLED display 160. When the third switch button is turned on, power 5 Volt is supplied to the circuit.

The activation of the lasers may be observed from the back of the device through the ventilation slots (not shown). The technical effect of the third switch button is to allow the user to interact with the user's electromagnetic field being near the first laser or approaching the device just before the second laser is turned on by the motion sensor. When the first laser is on and when the second laser is on, interactions with the human electromagnetic field occur in light beams of higher intensity and radiation density within the laser beam, which normalizes the characteristics of this field, and which allows the user to more actively develop concentration on the events that ensure a target, such as eternal life and, therefore, to quickly implement these events. This effect is further enhanced by the operation of the LED-lights.

It is also possible to remotely monitoring the device via the Internet. The above-mentioned effect is then realised on the most remote participants or users in the presence of the electromagnetic field due to the radiation of human thought and the parameters of the electromagnetic field of a user associated with the electromagnetic field of the planet.

Additionally, an OLED display can be turned on in the mode of reading number series. This is made by clicking on the large button 170 to the right of the OLED display. The LED-light located on the front panel of the device on the right side pulsates during the mode of reading number series with a frequency and intensity corresponding to the digits on the display. The digits or number series may be read from a SD-card inserted in a SD-card adapter in the front of the device 100.

By using the number series on the SD card, concentrations can be carried out with the desired control at the required level. Number series may be periodically added to the SD card. The number series recorded on the SD card is not deleted during factory assembly of the device. Specific number series may ensure the development of concentrations of eternal life for the user. Clicking the button on the right of the OLED-display will access the file from the SD-card. The numbers recorded on the SD card appear then on the screen.

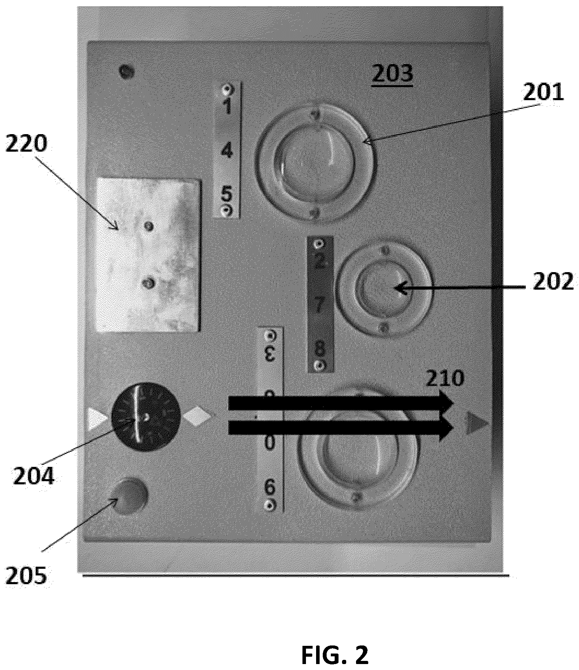

In FIG.2, on the upper surface of the device body or lid, there is a compass 204 with a mark for the location of the compass needle parallel to the beams of the lasers 210 located inside the device 200. The compass 204 allows determining the angle between the geographic North-South direction and the beam direction of the laser beams 210 which each is indicated by an arrow.

The lasers transmit a beam of red light to a large lens located inside the device. The beams are directed from the front panel to the rear wall strictly parallel to the side plane of the device. The lasers turn on activating the third switch button. One of the lasers is constantly on, and the other is connected to a motion sensor. In the absence of the user, this laser turns off 30 seconds after turning on and turns on when approaching the device at a distance of less than 2 meters.

The first laser beam 210 operates as a static wave of reality dispersing through the lens 450 into infinity, into the eternal environment. The dynamic reality wave function operates from a second laser beam also inside the device which is activated by a motion sensor 203. The second laser beam, which is activated by a motion sensor forms with the first laser beam a space between these laser beams in which an increase in intensity of thought radiation occurs due to the scattering and reflection of the laser beams in a large lens inside the device. When one laser beam operates, this effect is realized inside the lens in reflected and scattered sections of one laser beam.

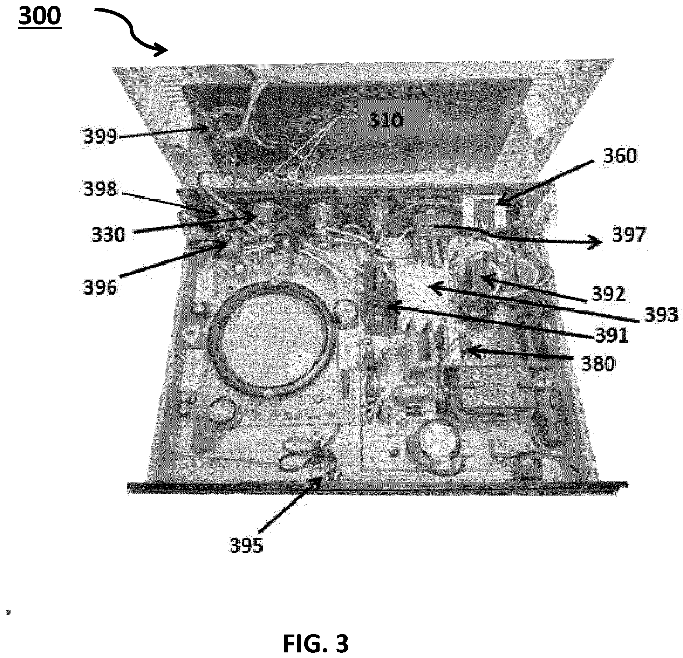

Fig. 3 shows the internal device of the present embodiment. On the lid, two lasers 310 are installed and at least one laser is connected to a sensor board 399 activating the motion sensor on the lid.

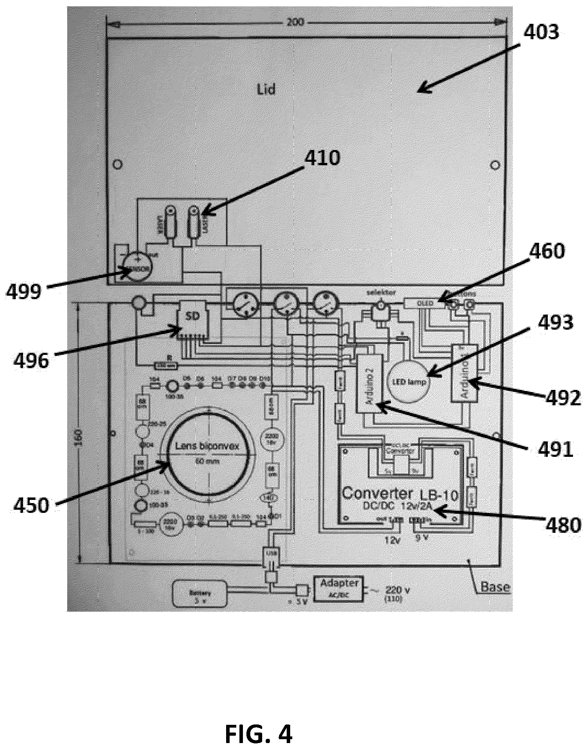

The device 300 is further configured to activate an artificial intelligence, Al, function programmed in two processing units 391, 392. These processing units may be of microcontrollers or similar for processing and transmitting information contained on the SD-card to the OLED-display or LED-light. These processing units 391, 392 are receiving electrical signals from the converter 380 which increase the input voltage from 5 V to the required 9-12V and transfers the signal to a larger converter 480 shown in FIG. 4 which converts the optical signal emitted by the optical emitting unit.

The Al function enables the device 300, depending on the activity of generation of thoughts by the user and depending on the degree of development of concentration on eternal life in respect to specific events, to independently switch off the operation modes of the device 300 and then, after a time period determined by the device 300, again switch on any of three operation modes. Accordingly, the procedure of activation of this artificial intelligence function was developed.

Specifically, the first processing unit 391 process and transmits information on SD-card to the display and the second processing unit 392 process and transmits information on the SD-card to a LED-light 398, in the form of light pulses of varying brightness and duration. The read information, containing number series, is then transmitted to the LED, which, depending on what numbers are contained in the card file, changes the pulsation (glow) mode. Each number corresponds to its own pulsation frequency and glow strength (brightness). The SD-card, in accordance with the wave synthesis process, realizes the transition of an electron to an infinite medium through periodic pulses of light in an LED-light through the dynamics of light scattering in space. Accordingly, the radiation of thought translates the information of the concentration goal corresponding to the number series into an infinite, eternal environment in which there is a systemic level of goal realization.

The device 300 may also comprise mini-buttons 170 for controlling the information that appears on the display 360. A short press on the top button moves the cursor from the top line down to the file we need (1.TXT). A short press on the lower button opens the file and the numbers written on the SD-Card appear on the display 360.

Further, the device 300 provides the capability to combine three modes of operation, thereby creating better concentration. The switch buttons 330 are connected to a switch 9-pin 397 for switching the signals from the SD-card to any of the processing units.

The output from the processing units containing a series of numbers for developing concentration is monitored in an OLED-display 360 but it could be any other display as a LED-display or LCD display.

There is also an SD-adapter or SD module 396, where a SD-card may be read if inserted for transferring information contained on the SD-card to the processing units or microcontrollers. As explained above, the device has an operation mode that allows the device to read the number series from the SD-card. The number series displayed on the display, in accordance with the process of wave synthesis, allow a transition of an electron into an infinite eternal environment or medium via the SD-card and via the software of the processing units.

Accordingly, the radiation of thought transfers the information of the concentration target corresponding to the number series into an infinite, eternal environment in which there is a systemic level of goal realization.

Each operating mode of the device, in connection with the operation of artificial intelligence, is enhanced by an SD card. By using the numbers on the SD card, the user may perform concentration with the desired control at the required level. Number series may be periodically added to the SD card. The number series recorded on the SD card is not deleted during factory assembly of the device. It is also possible to add individual number series, or any other pre-selected number series.

When the device is in the SD-reading mode, the light-emitting diode, LED, light on the right front side of the device is lighted up by a signal LED-diode 398 and pulsates with a frequency and intensity corresponding to the read numbers.

On the rear side of the device, there is a USB connector 395 for connecting for example external power to the device from a 5 V source. Moreover, there are mini-connector-USBs which supply 5 V to the second processing unit 392 by connecting to its connector.

Fig. 4 discloses an overview of the main components of one of the embodiment of the present disclosure. The lens-biconvex 450 may be 60mm but could of other sizes. This figure shows the same components than in previous figures, such as the sensor board 499 and the laser 410 installed on the lid 403, the OLED display 460, the LED lamp 493, the processing units 491, 493 , a converter 480,etc. In this embodiment, the processing units used are Arduino 1 and 2. However, there are many other microcontrollers and microcontroller platforms available for physical computing. Some examples are Parallax Basic Stamp, Netmedia's BX-24, Phidgets, MIT's Handyboard, and many others offer similar functionalities. All of these tools allow programming in an easy-to-use package. Arduino also simplifies the process of working with microcontrollers, and offers some advantage for teachers, students, and interested amateurs over other systems.

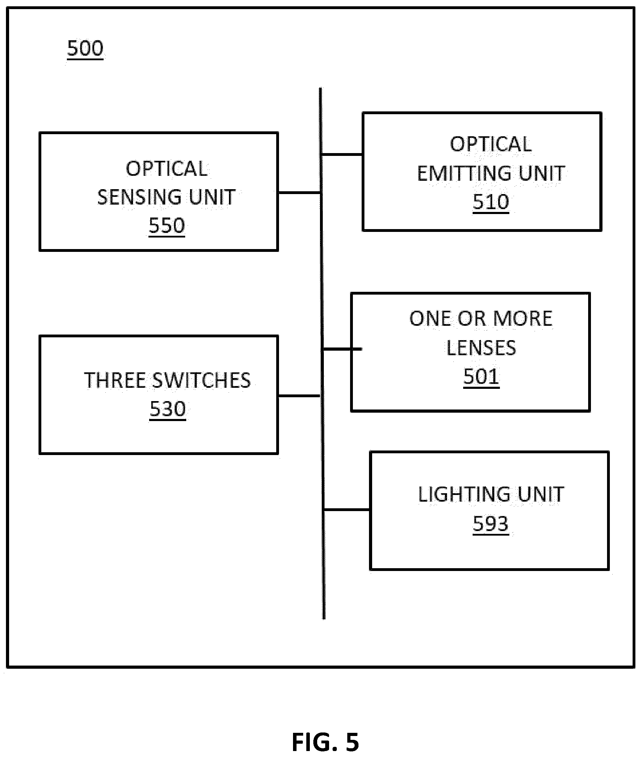

Fig. 5 is a block diagram showing various units of a three-mode device 500 for development of concentration, in accordance with certain embodiments. Specifically, the device 500 may include an optical sensing unit 550, optical emitting unit 510, one or more lenses 501, three switches 530, and a lighting unit 593. The device may include a housing and a lid. The one or more lenses 501 and the lasers may be disposed on the cover.

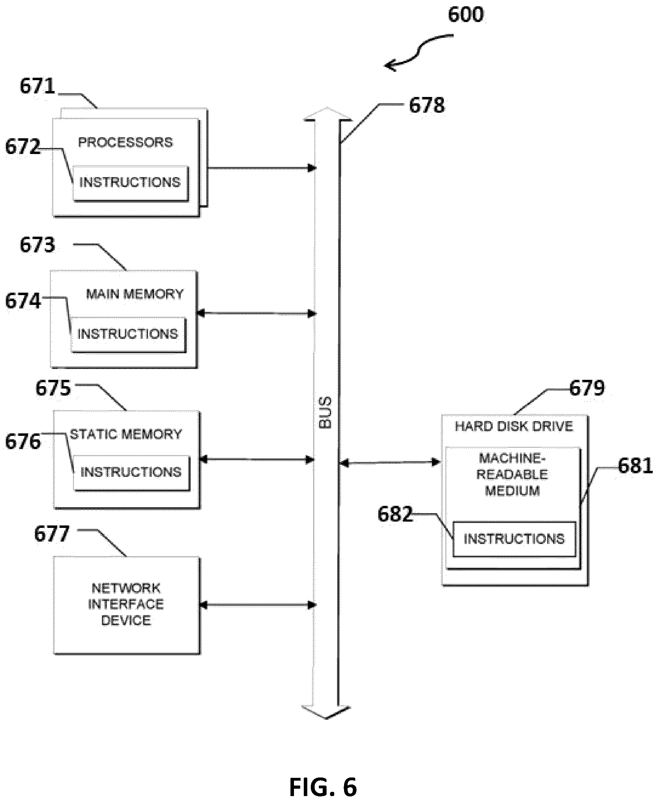

Fig. 6 shows a diagrammatic representation of a computing device for a device 600 according to the present disclosure within which a set of instructions for causing the device to perform any one or more of the methodologies discussed herein can be executed. In a networked deployment, the machine can operate in the capacity of a server or a client machine in a server-client network environment. This computer system includes at least one processor 671, main memory 673, static memory 675, which communicate with each other via a bus 678. The computing device may also include a network interface device 677. The hard disk drive 679 may include a computer-readable medium 681, which stores one or more sets of instructions 682 embodying or utilized by any one or more of the methodologies or functions described herein.

The instructions may be a software program such as attached in Annex 1 comprising a computer-implemented method of developing concentration loadable into the claimed device 100, 300. The instructions involve receiving an electrical signal converted by a converting unit from at least an outgoing signal. The outgoing signal is obtained from the optical sensing unit upon a biological signal associated with a plurality of electromagnetic fields provided by a user. In the next step the electrical signal is processed by a processing unit using artificial intelligence, Al, for outputting an optical signal emitted by the optical emitting unit, which is associated with the concentration of a user in the form of a blinking LED light. The Al may be any type of artificial algorithms.

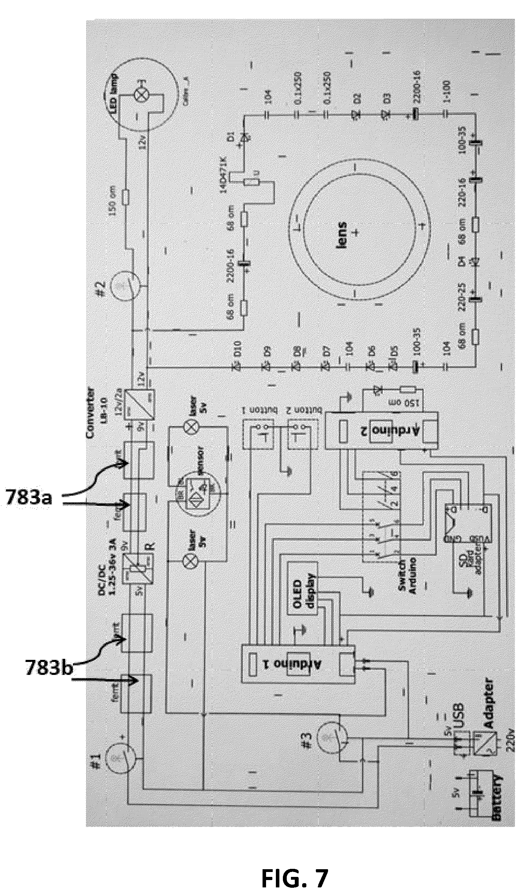

Fig. 7 shows an electrical scheme showing the electrical connections of all the components in the device. Components that are for instance connected between the converter LB-10 and the DC/DC converter but are not previously mentioned are ferrits 783 a, 783b.

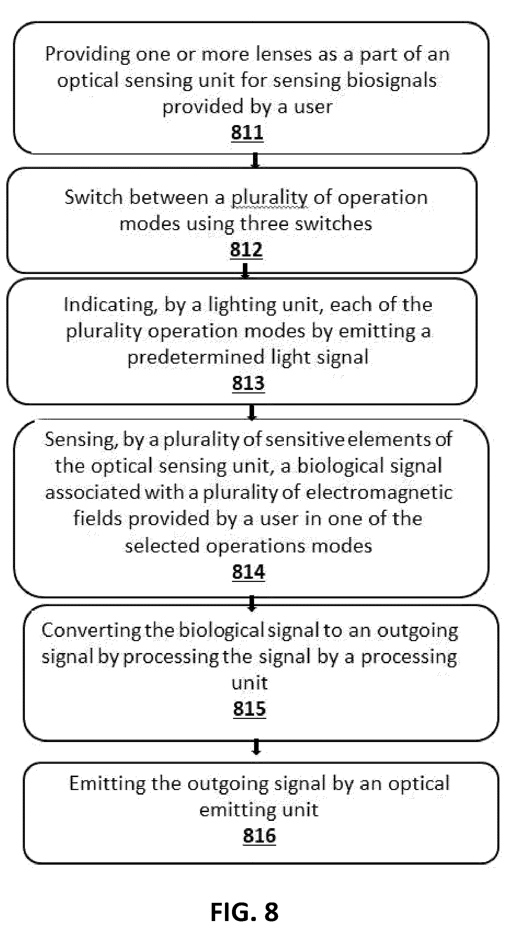

Fig. 8 depicts a flow diagram explaining every method steps of the method according to the present disclosure. The device 100-700 comprises an optical sensing unit and an optical emitting unit. The first method step 811 is therefore providing one or more lenses as a part of the optical sensing unit for sensing biological signals also called biosignals provided by the user. The second method step 812 is to switching between three operation modes using three switch buttons. The third method step 813 involves indicating, by a lighting unit or LED, each of the three operation modes by emitting a predetermined light signal. The fourth method step 814 involves sensing by a plurality of sensitive elements of the optical sensing unit, a biological signal associated with a plurality of electromagnetic fields provided by a user in one of the selected operation modes. The fifth method step 815 involves converting the biological signal to an outgoing signal by processing the signal by a processing unit to emit as a last step 816 the outgoing signal by an optical emitting unit. Whilst the invention has been described with respect to illustrative embodiments thereof, it will be understood that various changes may be made in the apparatus and means herein described without departing from the scope and the teaching of the invention. Accordingly, the described embodiments are to be considered merely exemplary and the invention or disclosure is not to be limited except as specified in the attached claims.

### Annex 1

The text of the sketch program which is in the device of the present disclosure displays number series from SD - Card to LED (LED - diode):

| | | |
|---|---|---|
| #define FILE_NAME | "1.txt" | // file to open |
| #define LED_PIN | 7 | // pin led is connected to |

// functions for every number 0-9

| | |
|---|---|
| #define NUM_0_FUNC | blinkLed(2, 500) |
| #define NUM_1_FUNC | blinkLed(3, 250) |
| #define NUM_2_FUNC | pulseLed(2, 100, 500) |
| #define NUM_3_FUNC | pulseLed(3, 10, 300) |
| #define NUM_4_FUNC | pulseLedUp(2, 100, 500) |
| #define NUM_5_FUNC | pulseLedUp(3, 10, 400) |
| #define NUM_6_FUNC | pulseLedUp(4, 10, 300) |
| #define NUM_7_FUNC | pulseLedDown(2, 100, 1000) |
| #define NUM_8_FUNC | pulseLedDown(3, 10, 750) |
| #define NUM_9_FUNC | pulseLedDown(4, 10, 500) |

/*
* blinkled(num, delay) - just blinking. Num - number of blinks, delay - delay between blinks (ms)
   *
* pulseLed(num, delay, speed) - pulsates (smoothly flares up and fades out)
* pulseLedUp(num, delay, speed) - pulsates upward (flares up smoothly, then goes out abruptly)
* pulseLedDown(num, delay, speed) - pulsates down (lights up sharply and goes out smoothly)
* num - number of blinks, delay - delay between blinks (ms), speed - speed at which it lights up and goes out (the higher the number, the slower) */

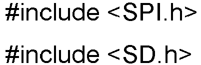

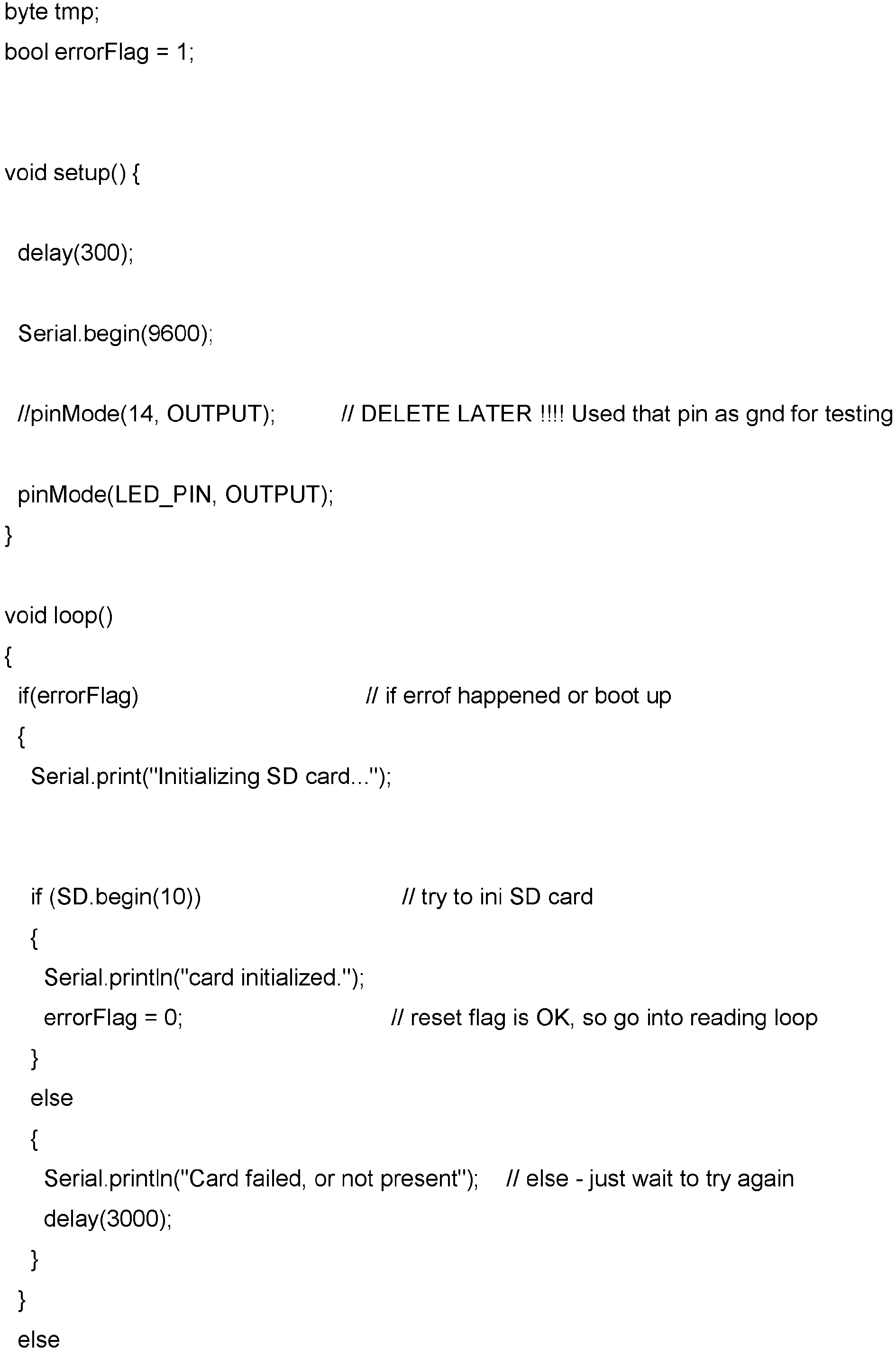

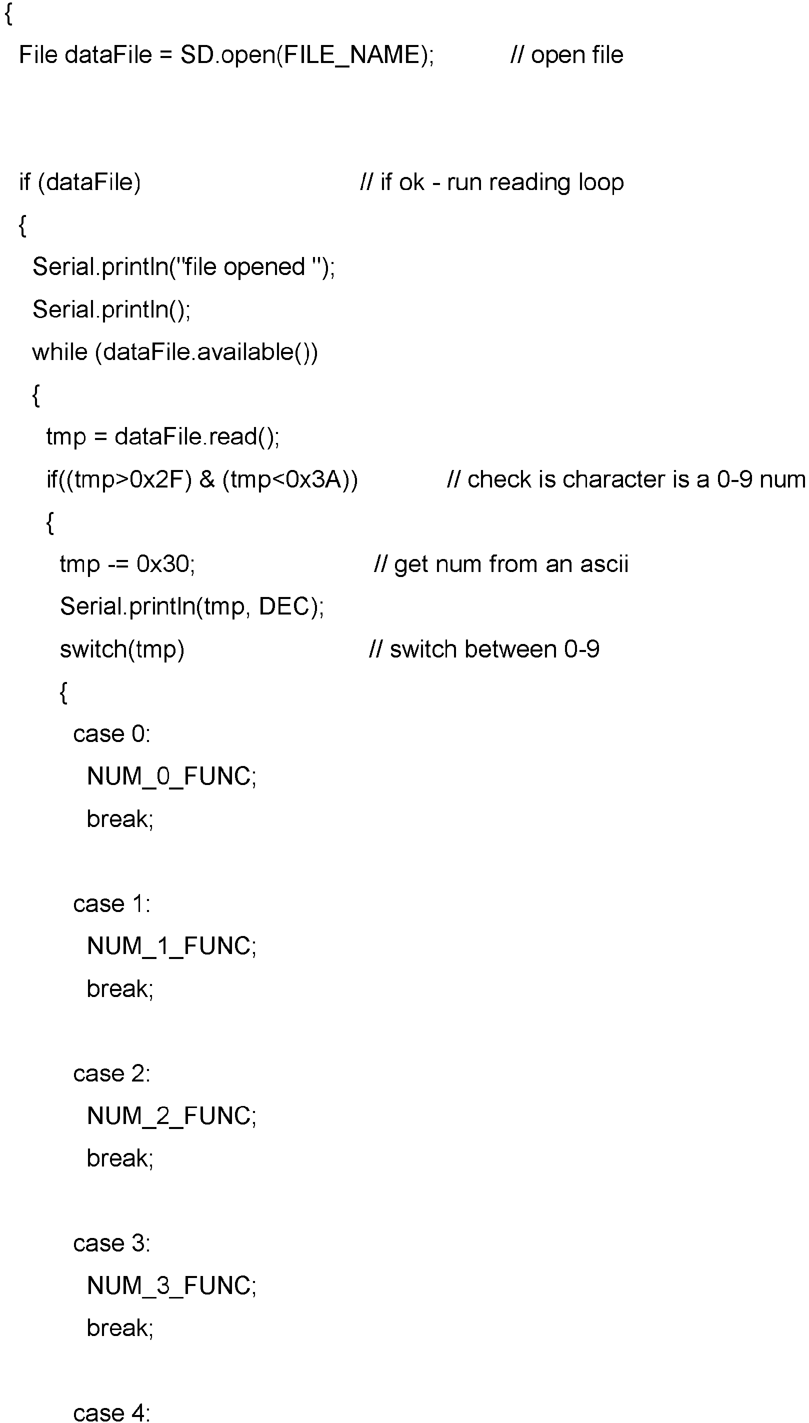

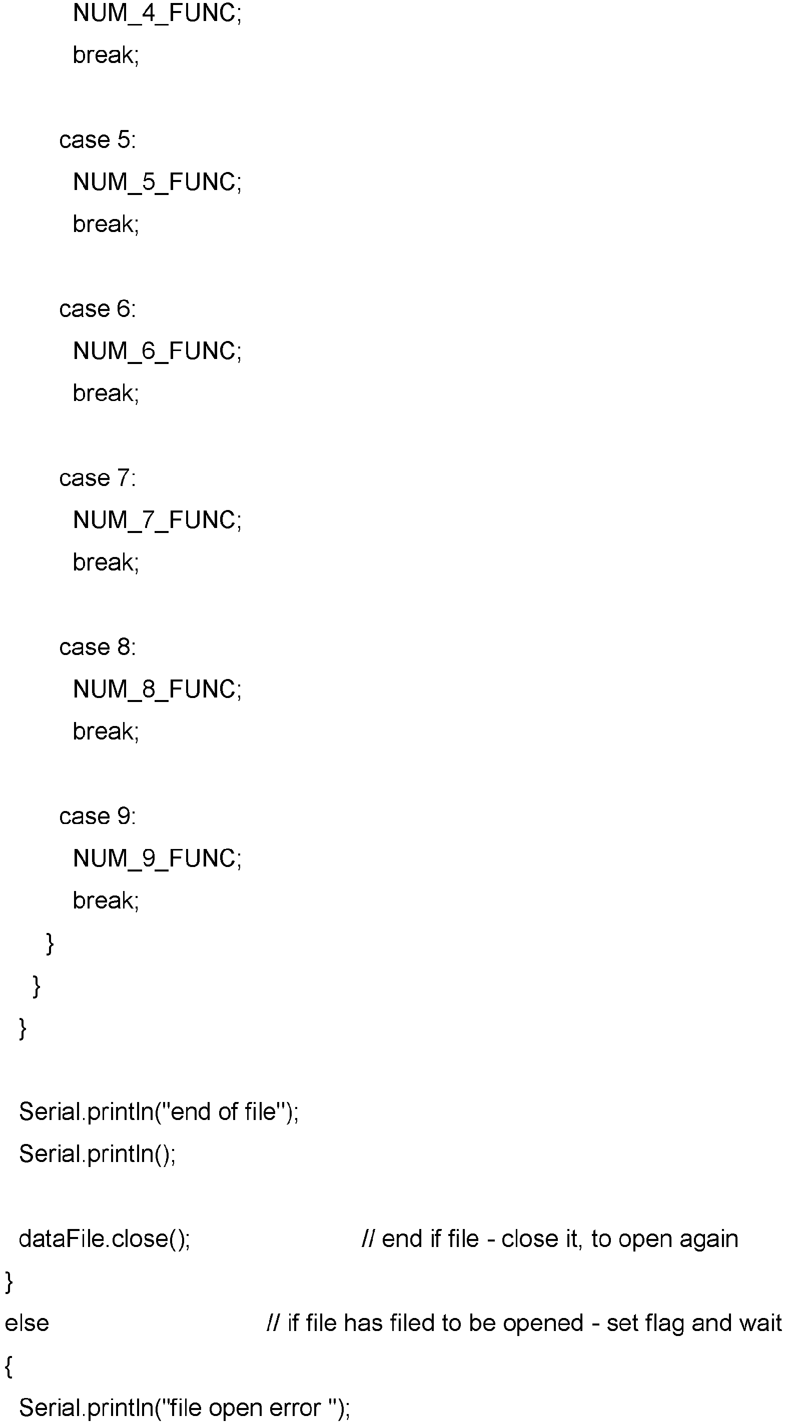

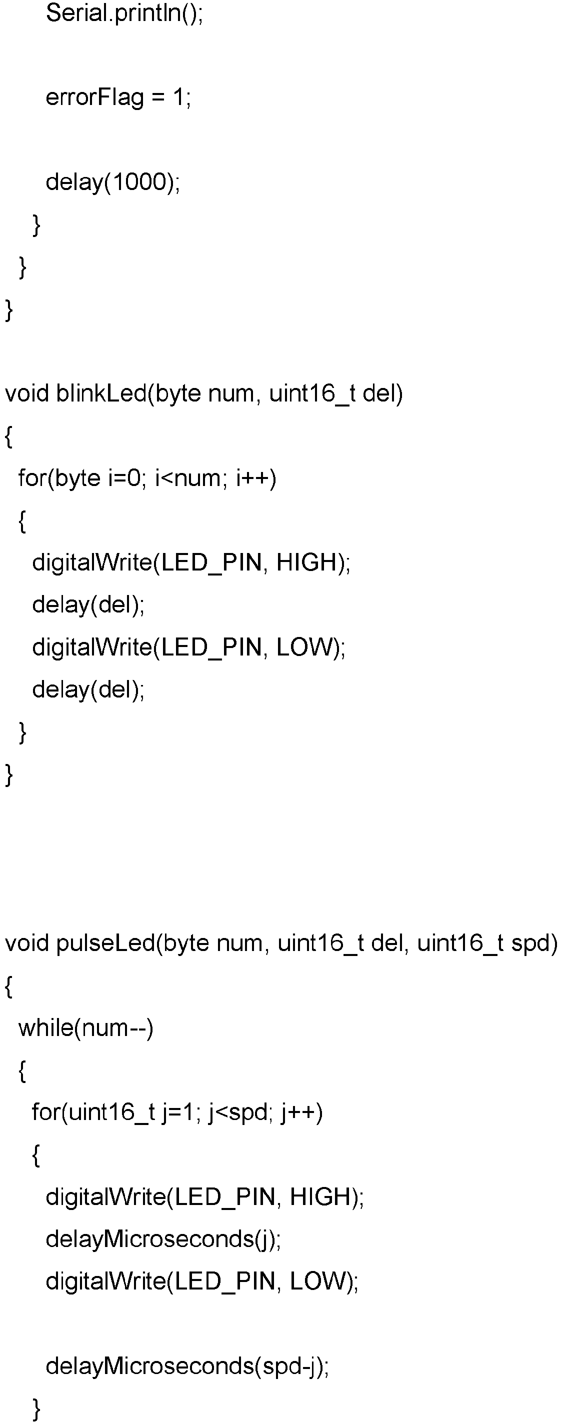

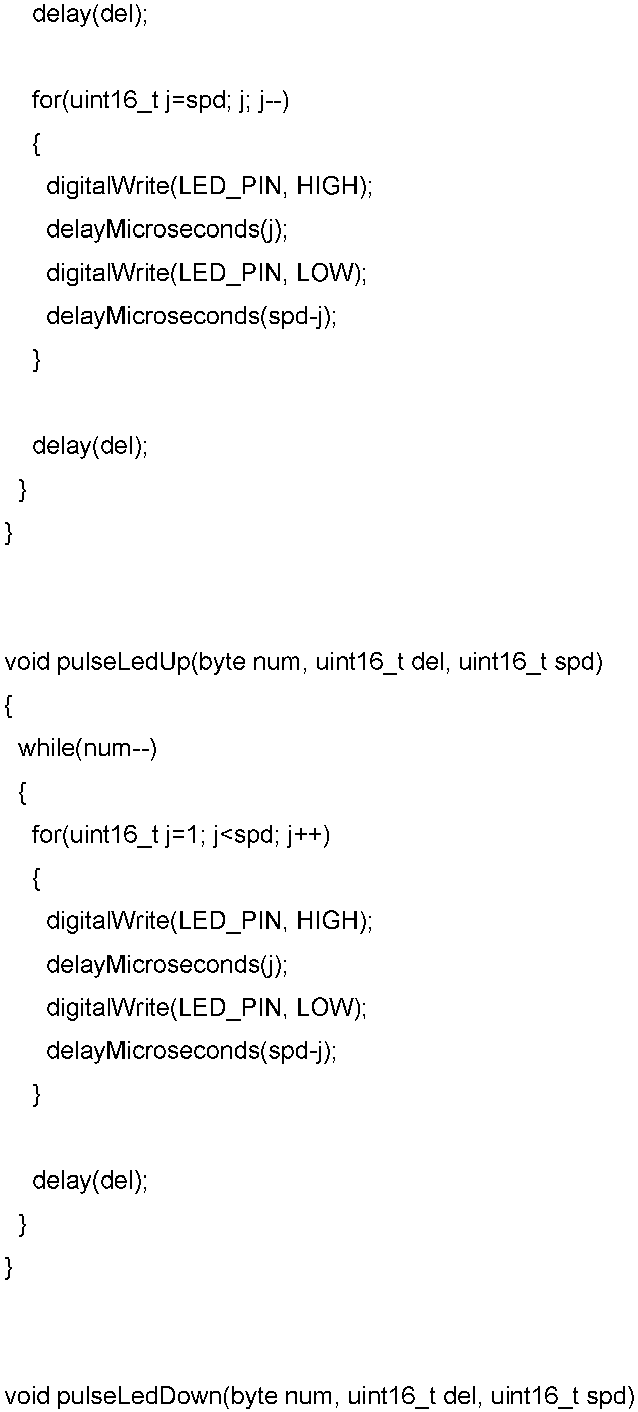

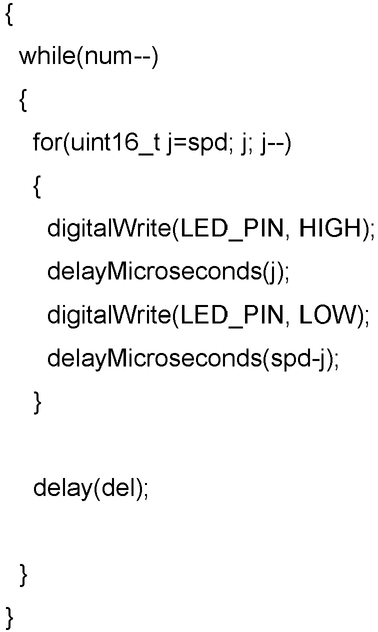

## Claims

1. A device (100) for development of concentration, the device (100) comprising:
- an optical sensing unit, the optical sensing unit comprising a plurality of one or more lenses (201, 202) capable of holding sensitive elements, wherein the plurality of sensitive elements are configured to sense a biological signal provided by a user in at least three operational modes, the signal being associated with a plurality of electromagnetic fields; and an outgoing signal is obtained based on the biological signal and the plurality of electromagnetic fields;
- an optical emitting unit configured to emit the outgoing signal; wherein the optical emitting unit emits the outgoing signal in a form of at least an optical signal;
- three switches for switching between the plurality of operation modes;
- a plurality of lightning units configured to indicate each of the plurality of operation modes by emitting a predetermined light signal;
**characterized by** further comprising:
- at least two lasers placed inside the device, wherein a first laser is constantly turned on during a second operation mode representing the emittance of a static light signal by one of the plurality of the lightning units; the second laser being connected to a motion sensor capable of turning on and off when a user is in a range proximity and representing the emittance of a repetitively-pulsed light signal by another lightning unit; and
- a processing unit for processing information from at least one of a motion sensor, a SD-card, a laser, a DC/DC converter, a switch selector, and a USB-adapter, by using artificial intelligence.

2. The device of claim 1, further **characterized by** comprising a power source in communication with the optical sensing unit and the optical emitting unit.

3. The device of any previous claims, **characterized in that** the plurality of sensitive elements are spherical.

4. The device of any previous claims, further **characterized by** comprising a housing and a lid.

5. The device of claim 4, wherein the device is **characterized by** comprising a plurality of numbers or letters placed on one of a housing and a cover, wherein the numbers or letters are symbols for focusing concentration of the user.

6. The device of claim 5, wherein a first set of numbers is **characterized by** including digits 1, 4 and 5 and a second set of numbers includes digits 2, 7, 8 and 9, 0, 6, 3.

7. The device of claim 4, **characterized in that** the one or more lenses (201, 202) are disposed on the lid.

8. The device of any previous claims, **characterized in that** the optical emitting unit includes an optical lens.

9. The device of any previous claims, further **characterized by** comprising a converting unit configured to convert the outgoing signal into an electrical signal.

10. The device of any previous claims, **characterized in that** a SD-card reader is installed.

11. The device of claim 10, **characterized in that** an OLED screen is displaying the number series read from a SD-card read from the SD-card adapter.

12. The device of any of previous claims, **characterized in that** a compass is installed on a lid of the device for directing the laser beams in a specific direction.

13. The device of any of previous claims, **characterized in that** a USB connector is installed on the rear of the device.

14. The device of claims 10 or 11, **characterized in that** there is provided LED-lights for displaying number series from a SD-card in the form of light pulses.

15. A method for development of concentration, the method comprising the steps of:
- providing one or more lenses for focusing concentration of a user, the one or more lenses being part of an optical sensing unit, the optical sensing unit and an optical emitting unit are comprised in the device according to any of claims 1-14;
- switching between a plurality of operation modes using three switches;
- indicating, by a lightning unit, each of the plurality operation modes by emitting a predetermined light signal; the method is **characterized by**
- sensing, by a plurality of sensitive elements of the optical sensing unit (210), a biological signal provided by a user in at least three operational modes, the signal being associated with a plurality of electromagnetic fields; and obtaining an outgoing signal based on the biological signal and the plurality of electromagnetic fields;
- converting the biological signal by a converting unit to obtain an outgoing signal; and
- processing the outgoing signal by a processing unit to obtain an optical signal generated by the optical emitting unit.

16. The method of claim 15, is further **characterized by** comprising the step of providing a power source, wherein the power source is in communication with the optical sensing unit and the optical emitting unit.

17. The method of any claims 15-16, **characterized in that** the plurality of sensitive elements is spherical in the sensing step.

18. The method of any of claims 15-17, **characterized in that** the optical emitting unit includes an optical lens.

19. A computer-implemented method of developing concentration loadable into the device according to any of claims 1-14, comprising the steps of:
- receiving an electrical signal converted by a converting unit from at least an outgoing signal; the optical signal is obtained from the optical sensing unit upon a biological signal associated with a plurality of electromagnetic fields provided by a user; and
**characterized by**
- processing the electrical signal by a processing unit using artificial intelligence for outputting an optical signal generated by the optical emitting unit associated with the concentration of a user.
